# EUROPEAN PATENT SPECIFICATION

(11) **EP 2 687 107 B1**
(45) Date of publication and mention of the grant of the patent: **14.09.2016**
(21) Application number: 12760379.3
(22) Date of filing: 10.04.2012
(51) Int. Cl.: A23L 33/105, A23L 33/175, A23L 21/20, A61P 15/10, A61K 45/06, A61K 35/64, A61K 31/198

(54) **BIOLOGICALLY ACTIVE FOOD ADDITIVE FOR THE PROPHYLAXIS OF ERECTILE DYSFUNCTION IN MEN**
BIOLOGISCH WIRKSAMER NAHRUNGSMITTELZUSATZSTOFF ZUR PROPHYLAXE DER EREKTILEN DYSFUNKTION BEI MÄNNERN
ADDITIF BIOACTIF POUR LA PRÉVENTION DE TROUBLES ÉRECTILES CHEZ L'HOMME

(30) Priority: 17.03.2011 RU 2011110120
(43) Date of publication of application: 22.01.2014
(73) Proprietor: Obshestvo S Ogranichennoj Otvetstvennostju "PARAFARM", Penza 440026 (RU)
(72) Inventor: TRIFONOV, Vyacheslav Nikolaevich, Zarechny Penzenskaya obl. 442960 (RU); ELISTRATOVA, Julia Anatoljevna, Penza 440026 (RU); ELISTRATOV, Konstantin Gennadievich, Penza 440061 (RU); KURUS, Natalia Vyacheslavovna, Penza 440061 (RU)
(74) Representative: Engel, Christoph Klaus
(86) International application number: PCT/RU2012/000271
(87) International publication number: WO 2012/128672

(56) References cited:
- WO-A1-01/19370
- WO-A1-02/102163
- CA-A1- 2 793 071
- CN-A- 101 444 590
- KR-A- 20030 049 063
- RU-C1- 2 390 270
- RU-C1- 2 412 616
- US-A1- 2002 037 862
- US-A1- 2005 220 905
- KHISMATULLINA N Z ET AL: "Passages", 1 January 2005 (2005-01-01), APITERAPIYA, MOBILE, PERM; RUSSIA, PAGE(S) 158-159, XP008169096, ISBN: 5-88187-263-0 * page 158 *
- KHISMATULLINA N.Z. APITERAPIIA.PERM, MOBILE 2005, page 158, XP008169096

## Description

This invention relates to a food processing industry, precisely to biologically active dietary additives (BAA) for the prevention of male erectile dysfunction which is based on the natural ingredients.

A BAA that contains a drone brood in amounts of 1-70 mass% - RU 2390270 C1, 27.05.2010 is known. A disadvantage of the known remedy is a long term development of therapeutic effect (up to 2 weeks).

There is a remedy for prevention of erectile dysfunction that contains L-Arginine in amounts of 50-80 weight fractions KR 20030049063 A, 25.06.2003). A disadvantage of the known remedy: all the pharmacological effects of L-arginine are associated with its converting within the human body into molecules of nitric oxide (NO). A nitric oxide in its turn, takes part in control action of blood circulation, immunologic and neural mediated functions, hepatic functions, blood coagulability and sexual function. A great amount of L-Arginine is needed in order to achieve a therapeutic effect - from 2.8 g. to 4 g. (Russian Encyclopedia of Biologically Active Dietary Supplements, Moscow, «GEOTAR-Media», 2007). This amount is now invalid for the release of BAA as a tablet or capsule, as it is impossible to produce especially to swallow a big tablet or capsule. The second major disadvantage of the known remedy is the fact that L-Arginine is not an indispensable amino acid and also can be received from regular food. Therefore, there is a risk that N0 formed in large quantities can be regenerated into the very long-lived toxic peroxynitrates anion. Unlike N0, peroxynitrates anion stimulates the capture of calcium by the mitochondria, hence dissociating the processes of tissue respiration and oxidative phosphorylation, which ultimately leads to a drop in the energy potential of nerve cells with all the consequences.

The research of a technical level in this matter demonstrated that big amount of medicinal products which promote the elimination of erectile dysfunction are known.

There is a famous medicinal product VIAGRA which is on the basis of such medicinal substance as Sildenafil citrate with a therapeutic dose of 25 mg. Side effects are: On the part of cardiovascular system: headache, hot flushes and phlebarteriectasia. On the part of central nervous system: dizziness, elevation of muscle tone and insomnia. On the part of gastrointestinal tract: dyspepsia, diarrhea and nausea. On the part of musculoskeletal system: myodynia and joint pain. On the part of respirator system: stuffiness in nose, pharyngitis, rhinitis, sinusitis, respiratory passage infection and respiratory distresses. On the part of sensory organs: a light change of visual sense modality (color perception, clearness), and conjunctivitis. On the part of skin integument: hives. On the part of urogenital system: urinary tract infections and dysfunction of prostatic gland. Other: asthenia, pain (including stomach and back), flu symptoms, and quite seldom -cases of persistent erection of penis (priapism).

Medicinal product Cialis which is on the basis of such medicinal substance as Tadalafil with a therapeutic dose of 5 mg is known. Side effects are: Body in whole: asthenia, facial oedema, fatigue and pain. On the part of cardiovascular system: stenocardia, chest pain, hypotension, hypertension, myocardial infarction, orthostatic hypotension, heartbeats, syncopal conditions and tachycardia. On the part of gastrointestinal tract: a change of liver function tests, diarrhea, xerostomia (dry mouth), dysphagia, esophagitis, gastroesophageal reflux, gastritis, the increase of a level of gamma-glutamyl transpeptidase, liquid liquid bowel habits, nausea, pain in upper stomach and vomiting. On the part of musculoskeletal system: arthralgia and neck ache. On the part of central nervous system: dizziness, elevation of muscle tone and insomnia, papaesthesia, drowsiness and vertigo. On the part of respirator system: labored breathing (dyspnea), nasal hemorrhage and pharyngitis. On the part of skin integument: hives, itch and hyperhidrosis. On the part of visual organs: impairment of vision, a change of chromatic vision, conjunctivitis (also including conjunctival hyperemia), ophthalmalgia, a rise of lacrimation and palpebral oedema.

The action of the specified medications is based on the physiological erection mechanism which involves the release of nitric oxide in cavernous bodies of penis in a course of sexual stimulation.

Objective of the invention is to provide the agent that meets the status of biologically active dietary additives (BAA), wherein the counterindications of medical agents mentioned above are eliminated, as well as the creation of an agent, with consideration of known prophylactic agents, not having the disadvantages of known prototypes:
1) the duration of the therapeutic effect
2) the potential overdosing,
3) the inability to prepare this means in the form of tablets or capsules because of the heavy weight of the mixture.

BAA supposed to include a minimum amount of pharmacopoeial ingredients with the most pronounced healing properties regarding erectile dysfunction with a minimum of counter indications and side effects.

At intake BAA must provide maximal preservation of the sum of biologically active substances; have easy to use and storage consumer shape, and possess a high manufacturing effectiveness in conditions of mass production.

The result achieved in use of this invention consists of reception of a pre-dosed agent respondent to the BAA status and consisting of a minimum number of highly efficient ingredients, cumulative effect of which, on the one hand, ensures an effective action against erectile dysfunction, and on the other hand has an extended range of preventive and revitalizing effects on the body.

Technological characteristics of BAA manufacturing do not cause complexities at their mass production, and the consumptive form provides a body perception of total amount of biologically active substances and the comfort at intake and storage.

Essence of the invention is in the provision of the biologically active dietary additive for the prevention of erectile dysfunction, which, in accordance with the invention, contains L-Arginine, as well as drone brood at the following correspondence of the ingredients in mass percent (%): L-Arginine 50% - 96.2 %, drone brood 3.8%-50%.

Declared BAA is produced in powdered, pelleted or capsular form, and also might be in a form of aqueous alcoholic extract and some forms on the basis of this extract, more specifically: powder, tablets and capsules. In order to solve an assigned task it was suggested to enhance a positive therapeutic effect of L-Arginine with an additional induction of drone brood as a donator of gamic entomological hormones: prolactin, estradiol, progesterone, testosterone - having a stimulating effect on the male androgenic functions. A drone brood packed with vitamins and hormones which are not hormone-substitutes is highly effective in the case of endocrine profile disorders. There is a possibility to achieve a harmonious functioning of all human bodily organs and systems. At the implementation of the assigned task, we found out a synergistic effect in regard to the improvement of male sexual function on account of a join usage of the components of suggested additive.

Effects of drone brood and L-Arginine are differently directed and their separate appliance is less efficient than their combined usage. It has the following explanation:
L-Arginine stimulates the blood flow to the area of lower pelvis, increases the strength and duration of blood filling of the male genitals that encourages the erection appearance. However, it is not always possible to achieve this effect. This is due to the fact that in many men with erectile dysfunction the process of biochemical converting of L-Arginine into N0 is impaired. There is a need for so called "conductors" in order to avoid this - other amino acids and minerals, in particular zinc, magnesium, potassium, choline, oleic, linoleic, linolenic and other, polyunsaturated fatty acids, as well as vitamins A, C, E - which are contained in drone brood.

The effect of drone brood is connected with entomological hormones (human prohormones) and predominantly with testosterone, the maintenance of which in the body is essential condition to stimulate the nerve ganglions of spinal cord, which control the erection. However, as it is mentioned above, the use of drone brood by itself also gives a little effect due to the slow development of therapeutic effect. There is a need for so called "starter" - nitrogen oxide. It is absent in drone brood, but it can be developed from L-Arginine.

As part of the study it was found that the joint use of drone brood and L-Arginine in one agent in declared proportions gives the over cooperative effect on account of speeding up the erection appearance. In this case L-Arginine is a catalyzer of the process or its "starter".

### Example of the product

L-Arginine - 250 mg, drone brood - 50 mg. Taking into account the counterindications of active materials of the declared agent, their reciprocal synergetic influence on one another, as also considering the fact that the tablet or capsule which size is more than 1200 mg is impossible to swallow (gag reflex), it seems appropriate to establish the range of declared agent as following: L-Arginine in a rate of 250 mg to 1160 mg, drone brood in a rate of 40 mg to 950 mg. Prepared powder blend is pelleted or capsulated. For the prevention of erectile dysfunction BAA in compound of L-Arginine - 250 mg, drone brood - 50 mg is taken by 2 tablets two times per day during one month out of food intake. An interval during the intake is one month. Indicated technical result is verified by the study conducted in the medical center "Secrets of Longevity" ( 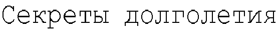 ) Penza, which demonstrated a high performance of declared remedy in comparison with control groups. The study included 15 men with the diagnosis of erectile dysfunction and chronic prostatitis, at the age from 30 to 50 years old (average age is 43). Duration of erectile dysfunction was from 6 months to 3 years (average duration is one year). Consent was received from all males participated in the study.

The experimental group (10 patients) was appointed to the proposed agent by 2 tablets twice a day in the morning hours. The first experimental group (10 patients) has been getting L-Arginine - 250 mg, 2 tablets 2 times per day in the morning hours.

The second experimental group (10 patients) has been getting drone brood - 50 mg, 2 tablets 2 times per day in the morning hours. Patients' examination involved the checkup, collection of complains in accordance with the symptoms scale 1P88, and questionnaire of International Index of Erectile Function (IIEF), with consideration of objective factors: ultrasound investigation of prostate gland, clinical blood analysis, urine test, including the results of prostate gland palpation. Patients' examination has taken place before and after the agent prescription.

An efficiency of the declared agent was estimated by the change of IIEF's exponents. Within a week, prior to and during the course of agent intake, patients were keeping a diary of disease estimation, and they recorded the frequency and degree of sexual attraction, valuation of erection, frequency of sexual intercourse and their sensations in each case. The conducted analysis demonstrated a positive improvement of sexual function as a consequence of course intake of declared agent and improvement of the indicators of patient's sexual function, as well as the achievement of the better exponents in the experimental group in comparison with both control groups. The effect of the applied agent in the experimental group starts within 2-3 hours after a taking of the agent, irrespective of age and the extent of sexual disorder, providing full sexual relations. Thus, the overall satisfaction composed to 90%, sexual intercourse satisfaction was 90%, erectile function in 100%, orgasm marker, change of sensations - 80%, the number of ejaculations has grown up in 80% of cases, refractory period after the emission of seminal fluid has changed for 100% of patients, strengthening of sexual attraction (libido) was recorded in 100% of cases.

Among 100% of patients with the symptoms of chronic prostatitis, has been tagged a good effect on the prescription of the agent, namely the disappearance of discomfort at the time of urination and drawing pain in the perineum. According to the data from questionnaire a reduction in all symptoms of chronic prostatitis and increase of intensity and duration of sexual sensation and duration of sexual intercourse has been registered. There also was a change in the quality of erection and increase in the number of sexual intercourses per unit of time, which contributed to the higher sexual self-esteem. In consequence of the research the improvement of quality of sexual life and subjective state of the patients has been registered. There were no clinically significant changes while investigating a blood test on sexual hormones and no dimensional changes of the prostate. In this study side effects were not observed; the specified agent is well tolerated, secure and easy in use.

The effect of the applied agent in the first control group starts within 2-3 hours after a taking of the agent, irrespective of age and the extent of sexual disorder, not providing the full sexual relations. Thus, the overall satisfaction was 20%, sexual intercourse satisfaction - 20%, erectile function - 20%, orgasm marker, change of sensations - 20%, the number of ejaculations has grown up in 20%, refractory period after the emission of seminal fluid has not changed in the case of 100% of patients, strengthening of sexual attraction (libido) was recorded in 100% of patients. The effect of the applied agent in the second control group starts within 4-6 hours after a taking of the agent, irrespective of age and the extent of sexual disorder, providing the full sexual relations. Thus, the overall satisfaction was 60%, sexual intercourse satisfaction - 60%, erectile function - 70%, orgasm marker, change of sensations - 60%, the number of ejaculations has grown up in 40%, refractory period after the emission of seminal fluid has changed among 40% patients, strengthening of sexual attraction (libido) was recorded in 100% of cases. In the course of undertaken studies above synergistic effect was observed with the use of components in claimed correlation, in mass percent (%): L-Arginine 50 - 96.2 and drone brood 3.8 - 50.

It is possible to conclude on the basis of undertaken study that the declared BAA comes to be a highly effective remedy for the patients with erectile dysfunction with a chronic prostatitis, and in various factors it exceeds its certain compounds (1 and 2 experimental groups). The action of the applied BAA directed to:
- strengthening sexual attraction (libido), specifically, receiving of over the total effect;
- improvement of the erection quality;
- orgasm oxypathia, change of sensations;
- stimulation of the intensity and duration of sex sensations;
- increase of sexual intercourse satisfaction;
- rise of sexual self-esteem;
- reduction of chronic prostatitis symptoms. The specified

BAA can be used either regularly or occasionally on account of its safety, the lack of addiction and side effects, it is well tolerated, secure and easy in use.

## Claims

1. A biologically active food additive (additive, BAA) for use in prophylaxis of erectile dysfunction in men which is **characterized by** the content of L-Arginine and drone brood at the following correspondents of ingredients, in mass percent L-Arginine 50 - 96.2%, Drone brood 3.8 - 50%.

2. The biologically active food additive in accordance with claim 1 provided in a form of powder, tablet or capsule.

## Patentansprüche

1. Biologisch wirksamer Lebensmittelzusatzstoff (biologically active additive, BAA) zur Verwendung in der Prophylaxe von Erektionsstörungen bei Männern, wobei er **dadurch gekennzeichnet ist, dass** der Gehalt an L-Arginin und Drohnenbrut den folgenden Zutaten in Massenprozent entspricht
L-Arginin 50 bis 96,2%,
Drohnenbrut 3,8 bis 50 %.

2. Biologisch wirksamer Lebensmittelzusatzstoff gemäß Anspruch 1, wobei dieser als Pulver, Tablette oder Kapsel bereitgestellt wird.

## Revendications

1. Additif alimentaire biologiquement actif (additif, ABA) pour une utilisation dans la prophylaxie du dysfonctionnement érectile chez l'homme qui est **caractérisé par** la concentration en L-arginine et en cellules mâles aux correspondances d'ingrédients suivantes, en pourcentage en masse :
L-arginine 50 à 96,2 %,
Cellules mâles 3,8 à 50 %.

2. Additif alimentaire biologiquement actif selon la revendication 1, fourni sous la forme de poudre, de comprimé ou de capsule.
